# FASCICULE DE BREVET EUROPEEN

(11) **EP 1 929 999 B1**
(45) Date de publication et mention de la délivrance du brevet: **16.03.2011**
(21) Numéro de dépôt: 07291391.6
(22) Date de dépôt: 23.11.2007
(51) Int. Cl.: A61K 31/165, A61P 25/00

(54) **Utilisation de l'agomélatine pour l'obtention de médicaments destines au traitement du syndrome de smith magenis**
Einsatz von Agomelatin zur Herstellung von Medikamenten zur Behandlung des Smith-Magenis-Syndroms (SMS)
Use of agomelatine to obtain medication aimed at treating Smith-Magenis Syndrome

(30) Priorité: 24.11.2006 FR 0610296
(43) Date de publication de la demande: 11.06.2008
(73) Titulaire: Les Laboratoires Servier, 92415 Courbevoie Cedex (FR)
(72) Inventeur: Mocaer, Elisabeth, 92200 Neuilly sur Seine (FR); Fabiano, Agnès, 37129 Verona (IT)

(56) Documents cités:
- EP-A- 1 564 202

## Description

La présente invention concerne l'utilisation de l'agomélatine ou *N*-[2-(7-méthoxy-1-naphtyl)éthyl]acétamide de formule (I) : 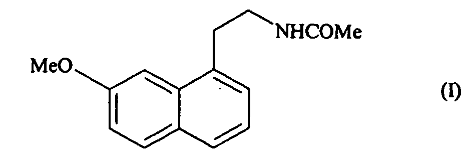 ainsi que ses hydrates, formes cristallines et sels d'addition à un acide ou à une base pharmaceutiquement acceptable, pour l'obtention de médicaments destinés au traitement du syndrome de Smith Magenis.

L'agomélatine ou *N*-[2-(7-méthoxy-1-naphtyl)éthyl]acétamide présente la double particularité d'être d'une part agoniste sur les récepteurs du système mélatoninergique et d'autre part antagoniste du récepteur 5-HT_{2C}. Ces propriétés lui confèrent une activité dans le système nerveux central et plus particulièrement dans le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'agomélatine, sa préparation et son utilisation en thérapeutique ont été décrits dans les brevets européens EP 0 447 285 et EP 1 564 202.

La demanderesse a présentement découvert que l'agomélatine ou *N*-[2-(7-méthoxy-1-naphtyl)éthyl]acétamide, ainsi que ses hydrates, formes cristallines et sels d'addition à un acide ou à une base pharmaceutiquement acceptable, possédait des propriétés intéressantes permettant de l'utiliser dans le traitement du syndrome de Smith Magenis, en combinaison avec l'acébutolol.

Décrit par Ann Smith et al. en 1982 (Smith A. C. M. et al, 1986, Am. J. Med. Genet., 24, 393-414), le Syndrome de Smith Magenis (SMS) est une maladie génétique rare due à une micro délétion chromosomique. Cette maladie particulièrement grave entraîne, chez l'enfant, l'apparition d'un syndrome dysmorphique, un retard mental notamment au niveau de l'acquisition du langage, une hyperactivité avec déficit de l'attention, une auto-agressivité associée à de graves troubles du comportement et du sommeil (Smith, A. C. M. et al., 1998, Am. J. Med. Genet., 81, 186-191).

A ces éléments il faut ajouter la détresse de l'entourage de l'enfant confronté à la maladie, qui non seulement doit accepter le handicap mais en plus se retrouve exposé à des conditions de vie extrêmes (nuits constamment perturbées, vigilance accrue de tous les instants, agressivité de l'enfant à contrôler...) menant le plus souvent à une dislocation totale de la cellule familiale.

L'incidence est de 1/25 000 naissances vivantes. Le diagnostic est basé sur les caractéristiques cliniques et confirmé par la mise en évidence de la délétion du chromosome 17 par un caryotype à haute résolution. Une inversion du rythme circadien de la mélatonine a été récemment mise en évidence et serait à l'origine des troubles du sommeil et des troubles du comportement (De Leersnyder H., 2006 Trends in Endocrinology and Metabolism, 17(7), 29-298).

S'il n'existe pas de traitement réellement satisfaisant et reconnu pour le SMS, les médicaments les plus utilisés actuellement sont les neuroleptiques, les hypnotiques, les psycho-stimulants, les antidépresseurs, les antipsychotiques et la carbamazépine pour contrôler les troubles du comportement. Ces traitements sont à l'origine de nombreux effets secondaires comme des troubles gastro-intestinaux, une prise de poids, des dyslipidémies, une dysfonction sexuelle, des dyskinésies tardives et un impact cardio-vasculaire (Richelson E. et al., 1999, J. Clin. Psychiatry, 60(10), 5-14; Trenton A. J. et al, 2003, CNS Drugs, 17 (5), 307-324; Freedman R. et al., 2003, New England Journal of Medicine, 343, 1738-1749). De plus, ces traitements n'ont aucune activité sur la désynchronisation de la sécrétion de la mélatonine, à l'origine des troubles majeurs du sommeil et de certains troubles du comportement dont l'impact est particulièrement perturbant pour l'enfant et sa famille.

A ce jour, la stratégie pour traiter la désynchronisation de la sécrétion de la mélatonine est une administration matinale d'antagonistes béta-adrénergique (acébutolol, propranolol) pour bloquer la sécrétion endogène de la mélatonine associée à une administration le soir de mélatonine exogène (De Leersnyder H. et al, 2003, J. Med. Genet., 40, 74-78). Cependant, la mélatonine reste dépourvue d'activité sur les troubles du comportement.

Ainsi la mise à disposition de nouveaux traitements pour cette pathologie orpheline de l'enfant est d'un intérêt majeur. En particulier, la mise au point d'un traitement permettant de pallier à la fois les troubles majeurs du sommeil et les troubles du comportement permettrait à l'enfant ainsi qu'à son entourage de retrouver une qualité de vie plus décente.

La demanderesse a présentement découvert que l'agomélatine, de par ses caractéristiques pharmacologiques est particulièrement bien adapté dans cette indication. L'agomélatine permet en effet d'agir à la fois sur les troubles du comportements et sur la resynchronisation des rythmes circadiens perturbés. De plus, l'agomélatine ne présente aucune interaction médicamenteuse et a un profil d'acceptabilité optimal : plus de 4000 patients ont été exposés à l'agomélatine au cours des essais cliniques réalisés et une excellente tolérance clinique et biologique a pu être observée.

L'invention concerne donc l'utilisation de l'agomélatine en combinaison avec l'acébutolol, ainsi que ses hydrates, formes cristallines et sels d'addition à un acide ou à une base pharmaceutiquement acceptable, pour l'obtention de compositions pharmaceutiques destinées au traitement du syndrome de Smith Magenis.

Particulièrement, l'invention concerne l'utilisation de l'agomélatine obtenue sous la forme cristalline II décrite dans la demande de brevet EP 1 564 202, pour l'obtention de compositions pharmaceutiques destinées au traitement du syndrome de Smith Magenis.

Les compositions pharmaceutiques seront présentées sous des formes convenant aux administrations par voie orale, parentérale, transcutanée, nasale, rectale, perlinguale, et notamment sous forme de préparations injectables, comprimés, comprimés sublinguaux, glossettes, gélules, capsules, tablettes, suppositoires, crèmes, pommades, gels dermiques.

Outre l'agomélatine et l'acébutolol, les compositions pharmaceutiques selon l'invention contiennent un ou plusieurs excipients ou véhicules choisis parmi des diluants, des lubrifiants, des liants, des agents de désintégration, des absorbants, des colorants, des édulcorants.

A titre d'exemple et de manière non limitative, on peut citer :
◆ *pour les diluants :* le lactose, le dextrose, le sucrose, le mannitol, le sorbitol, la cellulose, la glycérine,
◆ *pour les lubrifiants* : la silice, le talc, l'acide stéarique et ses sels de magnésium et de calcium, le polyéthylène glycol,
◆ *pour les liants* : le silicate d'aluminium et de magnésium, l'amidon, la gélatine, la tragacanthe, la méthylcellulose, la carboxyméthylcellulose de sodium et la polyvinylpyrrolidone,
◆ *pour les désintégrants* : l'agar, l'acide alginique et son sel de sodium, les mélanges effervescents.

La posologie utile varie selon l'âge et le poids du patient, la voie d'administration, la nature de l'affection et des traitements éventuellement associés et s'échelonne entre 1 mg et 50 mg d'agomélatine par 24 heures.

De manière préférentielle, la dose journalière d'agomélatine sera de 25 mg par jour, avec possibilité d'augmenter à 50 mg par jour.

### Composition pharmaceutique :

Formule de préparation pour 1000 comprimés dosés à 25 mg :

| | |
|---|---|
| N-[2-(7-méthoxy-1-naphtyl)éthyl]acétamide | 25 g |
| Lactose monohydrate | 62 g |
| Stéarate de Magnésium | 1,3 g |
| Povidone | 9 g |
| Silice colloïdale anhydre | 0,3 g |
| Cellulose sodium glycolate | 30 g |
| Acide stéarique | 2,6 g |

### Etude clinique :

Une étude exploratoire de phase II a été réalisée chez l'enfant présentant un syndrome de Smith Magenis. L'agomélatine 1-5 mg/kg a été coadministré avec de l'acébutolol 10 mg/kg, antagoniste β1 adrénergique. Les critères principaux d'analyse ont été des paramètres d'actigraphie enregistrés sur cinq périodes de 30 jours consécutifs, après 30 jours de traitement, après 3, 5 et 15 mois de traitement, ainsi que le questionnaire d'Achenbach permettant d'évaluer les troubles du comportement.
Les résultats obtenus montrent avec l'agomélatine une diminution de la fréquence et de la durée des réveils nocturnes, qui s'accompagne d'une réduction de la durée des siestes diurnes. Une amélioration clinique majeure a été constatée par un spécialiste de cette pathologie pour ces enfants traités par l'agomélatine : pour la première fois un sommeil calme et profond, des nuits moins fragmentées et un réveil moins tôt dans la matinée ont été enregistrés.
Des progrès réels au niveau du comportement ont également été observés. Ces effets majeurs se sont reportés sur les familles qui, suite aux conséquences très positives du traitement sur la vie familiale, ont requis, à titre compassionnel la poursuite du traitement.

## Revendications

1. Utilisation de l'agomélatine ou *N*-[2-(7-méthoxy-1-naphtyl)éthyl]acetamide ou un de ses hydrates, formes cristallines ainsi que ses sels d'addition à un acide ou à une base pharmaceutiquement acceptable, en combinaison avec l'acébutolol pour l'obtention d'un médicament destiné au traitement du syndrome de Smith Magenis.

2. Utilisation selon la revendication 1 **caractérisée en ce que** l'agomélatine est obtenu sous la forme cristalline II.

3. Compositions pharmaceutiques contenant de l'agomélatine ou un de ses hydrates, formes cristallines ainsi que ses sels d'addition à un acide ou à une base pharmaceutiquement acceptable, en combinaison avec l'acébutolol, avec un ou plusieurs excipients pharmaceutiquement acceptables, pour utilisation dans le traitement du syndrome de Smith Magenis.

4. Composition pharmaceutique selon la revendication 3 **caractérisée en ce que** l'agomélatine est obtenu sous la forme cristalline II.

5. Agomélatine ou N-[2-(7-méthoxy-1-naphtyl)éthyl]acétamide ou un de ses hydrates, formes cristallines ainsi que ses sels d'addition à un acide ou à une base pharmaceutiquement acceptable, en combinaison avec l'acébutolol pour utilisation dans le traitement du syndrome de Smith Magenis.

6. Forme cristalline II de l'agomélatine en combinaison avec l'acébutolol pour utilisations dans le traitement du syndrome de Smith Magenis.

## Claims

1. Use of agomelatine, or *N*-[2-(7-methoxy-1-naphthyl)ethyl]acetamide, or one of its hydrates, crystalline forms or addition salts with a pharmaceutically acceptable acid or base, in combination with acebutolol in obtaining a medicament intended for the treatment of Smith-Magenis syndrome.

2. Use according to claim 1, **characterised in that** the agomelatine is obtained in crystalline form II.

3. Pharmaceutical compositions comprising agomelatine, or one of its hydrates, crystalline forms or addition salts with a pharmaceutically acceptable acid or base, in combination with acebutolol, with one or more pharmaceutically acceptable excipients, for use in the treatment of Smith-Magenis syndrome.

4. Pharmaceutical composition according to claim 3, **characterised in that** the agomelatine is obtained in crystalline form II.

5. Agomelatine, or *N*-[2-(7-methoxy-1-naphthyl)ethyl]acetamide, or one of its hydrates, crystalline forms or addition salts with a pharmaceutically acceptable acid or base, in combination with acebutolol for use in the treatment of Smith-Magenis syndrome.

6. Crystalline form II of agomelatine in combination with acebutolol for use in the treatment of Smith-Magenis syndrome.

## Patentansprüche

1. Verwendung von Agomelatin oder *N*-[2-(7-Methoxy-1-naphthyl)-ethyl]-acetamid oder eines seiner Hydrate, Kristallformen sowie seiner Additionssalze mit einer pharmazeutisch annehmbaren Säure oder Base in Kombination mit Acebutolol für die Herstellung eines Arzneimittels zur Behandlung des Smith-Magenis-Syndroms.

2. Verwendung nach Anspruch 1, **dadurch gekennzeichnet, dass** Agomelatin in der Kristallform II vorliegt.

3. Pharmazeutische Zubereitungen enthaltend Agomelatin oder eines seiner Hydrate, Kristallformen sowie seiner Additionssalze mit einer pharmazeutisch annehmbaren Säure oder Base in Kombination mit Acebutolol, mit einem oder mehreren pharmazeutisch annehmbaren Hilfsstoffen zur Verwendung bei der Behandlung des Smith-Magenis-Syndroms.

4. Pharmazeutische Zubereitung nach Anspruch 3, **dadurch gekennzeichnet, dass** Agomelatin in der Kristallform II vorliegt.

5. Agomelatin oder *N*-[2-(7-Methoxy-1-naphthyl)-ethyl]-acetamid oder eines seiner Hydrate, Kristallformen sowie seine Additionssalze mit einer pharmazeutisch annehmbaren Säure oder Base in Kombination mit Acebutolol zur Verwendung bei der Behandlung des Smith-Magenis-Syndroms.

6. Kristallform II von Agomelatin in Kombination mit Acebutolol zur Verwendung bei der Behandlung des Smith-Magenis-Syndroms.
